(19) Europäisches Patentamt
European Patent Office
Office européen des brevets

(11) Veröffentlichungsnummer : **0 299 907 B1**

(12) **EUROPÄISCHE PATENTSCHRIFT**

(45) Veröffentlichungstag der Patentschrift :
**04.03.92 Patentblatt 92/10**

(51) Int. Cl.$^5$ : **C07C 69/757,** C07C 67/343

(21) Anmeldenummer : **88730142.2**

(22) Anmeldetag : **01.07.88**

(54) **Verfahren zur Herstellung von symmetrischen Bicyclo[3.3.0]octan-diondicarbonsäurediestern.**

(30) Priorität : **17.07.87 DE 3724187**

(43) Veröffentlichungstag der Anmeldung :
**18.01.89 Patentblatt 89/03**

(45) Bekanntmachung des Hinweises auf die Patenterteilung :
**04.03.92 Patentblatt 92/10**

(84) Benannte Vertragsstaaten :
**AT BE CH DE ES FR GB GR IT LI LU NL SE**

(56) Entgegenhaltungen :
**CHEMISCHE BERICHTE, Band 115, Nr. 4, 1982, Seiten 1437-1447, Verlag Chemie GmbH, Weinheim, DE; M. HARRE et al.: "Stereoselektive cyclopentanon-Anellierungen"**

(73) Patentinhaber : **SCHERING AKTIENGESELLSCHAFT Berlin und Bergkamen
Müllerstrasse 170/178 Postfach 65 03 11
W-1000 Berlin 65 (DE)**

(72) Erfinder : **Skuballa, Werner, Dr.
Olwenstrasse 13
W-1000 Berlin 28 (DE)**

EP 0 299 907 B1

Anmerkung : Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Die Erfindung betrifft ein neues Verfahren zur Herstellung von symmetrischen Bicyclo [3.3.0] octandiondicarbonsäurediestern. Optisch aktives 6a-Carba-prostacyclin und besonders einige davon abgeleitete Verbindungen besitzen als stabile Analoga des natürlich Prostacyclins ($PGI_2$) einen hohen therapeutischen Nutzen [R.C. Nickolsen, M.H. Town, H. Vorbrüggen: Prostacyclin-Analogs, Medicinal Research Reviews, Vol. 5, No. 1, S. 1-53 (1985)]. Die in dieser neueren Übersicht aufgeführten Synthesen sind lang und führen teilweise nur zu racemischen Carbacyclinen. Besonders aufwendig sind die Synthesen, die zu Carbacyclinen in der dem natürlichen $PGI_2$ entsprechenden absoluten Konfiguration führen. Das liegt daran, daß gut zugängliche, geeignete Ausgangsmaterialien achiral sind und die optische Aktivität erst im Syntheseverlauf in hierfür geeignete Zwischenstufen eingeführt werden muß.

Es ist bekannt, daß sich symmetrische, prochirale Dicarbonsäurediester von Prostacyclin- und Carbacyclin-Zwischenstufen in sehr guten Ausbeuten enantioselektiv zu den Monocarbonsäureestern verseifen und decarboxylieren lassen, wenn man hierfür Enzyme, insbesondere $\alpha$-Chymotrypsin, verwendet (DE 36 38 760.6).

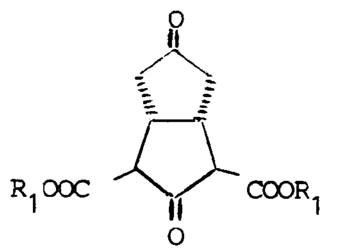

I

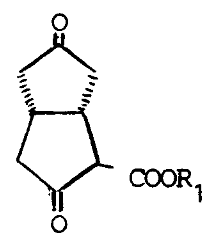

II

Der als Ausgangsmaterial für dieses Verfahren verwendete symmetrische Diester I wird nach Literaturmethoden aus 4-Acetoxy-2-cyclopenten-1-on und Acetondicarbonsäureester hergestellt. [V. Osterthun und E. Winterfeldt, Chem. Ber. 110, 146 (1977); M. Harre, P. Raddatz, R. Walenta und E. Winterfeld, Angew. Chem. 94, 496 (1982)]. In beiden Publikationen wird die Acetoxygruppe im 4-Acetoxy-2-cyclopenten-1-on als für die Umsetzung notwendige Fluchtgruppe hervorgehoben. Die Herstellung von 4-Acetoxy-2-cyclopenten-1-on erweist sich jedoch als besonders aufwendig und obendrein als unökonomisch. Man hat für dessen Synthese bis heute noch keine wirtschaftliche Synthese gefunden und mußte auf die kostspieligen Ausgangsmaterialien 4-Brom-2-cyclo-penten-1-on und Silberacetat zurückgreifen.

Es wurde nun gefunden, daß sich die oben genannten prochiralen, symmetrischen Dicarbonsäurediester I in sehr guten Ausbeuten aus dem leicht zugänglichen 4-Hydroxy-2-cyclopenten-1-on [P.G. Beraldi et al., Synthesis 1986, 781; K. Ogura et al., Tetr. Lett 1976, 759] oder 4-tert.-Butoxy-2-cyclopenten-1-on oder 4-Cumyloxy-2-cyclopenten-1-on [S. Takano et al., Chem. Pharm. Bull. 34 (8), 3445 (1986); S. Takano et al., Heterocycles 16, 605 (1981)] und Acetondicarbonsäureester herstellen lassen.

Die Erfindung eignet sich zur ökonomischen Herstellung der symmetrischen, prochiralen Diester der allgemeinen Formel I.

Die Erfindung betrifft somit ein Verfahren zur Herstellung von Bicyclo-[3.3.0]-octandicarbonsäurediestern der allgemeinen Formel I,

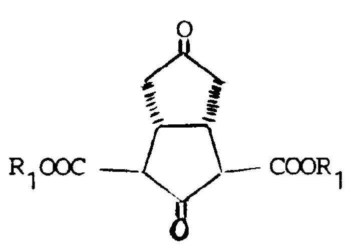

(I),

worin $R_1$ eine gerad- oder verzweigtkettige Alkylgruppe mit 1-10 C-Atomen darstellt, dadurch gekennzeichnet, daß man ein 4-Hydroxy- oder 4-Alkoxy-2-Cyclopenten-1-on der allgemeinen Formel III,

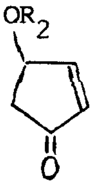

(III),

worin $R_2$ ein Wasserstoffatom oder eine gerad- oder verzweigtkettige Alkylgruppe mit 1-10 C-Atomen, die gegebenenfalls durch eine Phenylgruppe substituiert sein kann, mit Acetondicarbonsäurediester der Formel IV,

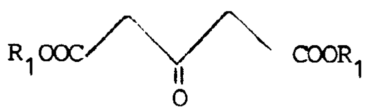

(IV),

worin $R_1$ die oben genannte Bedeutung hat, in Gegenwart einer Base umsetzt.

Als Base für das obige Verfahren können beispielsweise Kaliumcarbonat, Natriumcarbonat oder ein tertiäres Amin wie beispielweise Diisopropylethylamin dienen. Die Umsetzung wird bei -60 °C bis + 60 °C, vorzugsweise 0 °C bis 40 °C durchgeführt. Als Lösungsmittel oder als Lösungsmittelgemische können beispielsweise Methanol, Ethanol, Isopropanol, Methylenchlorid, Tetrahydrofuran u.a. verwendet werden.

Als gerad- oder verzweigtkettige Alkylreste mit 1-10 C-Atomen kommen die Rest Methyl, Ethyl, Propyl, Isopropyl, n-Butyl, Isobutyl, tert.-Butyl, n-Pentyl, Isopentyl, Neopentyl, n-Hexyl, Isohexyl, n-Heptyl, Isoheptyl, n-Octyl, Isooctyl, n-Nonyl, n-Decyl u.a. einfügen. Bevorzugt sind Alkylreste mit 1-6 C-Atomen, die durch einen Phenylrest substituiert sein können. Genannt seien z.B. Benzyl, Phenethyl, Cumyl etc.

Die nach dem erfindungsgemäßen Verfahren hergestellten Verbindungen der Formel I dienen zur Herstellung pharmakologisch wirksamer Prostacyclin-Derivate.

Die Verbindungen der allgemeinen Formel I können zur Herstellung pharmakologisch wirksamer Carbacyclin-Derivate eingesetzt werden [siehe hierzu R.C. Nickolson, M.N. Town und H. Vorbrüggen. Medicinal Research Review 5, 1 (1985) und P.A. Aristoff in Advances in Prostaglandin, Thromboxane and Leukotriene Research. Vol. 15 (1985)].

Ausgehend von 2.4-Bismethoxycarbonyl-bicyclo [3.3.0] octan-3.7-dion gelangt man beispielsweise in einer mehrstufigen Synthese zum Wirkstoff Iloprost. Enyntioselektive, enzymatische Verseifung, beispielsweise mit α-Chymotrypsin, liefert gemäß DE 36 38 760.6 den optisch aktiven Monoester IIa.

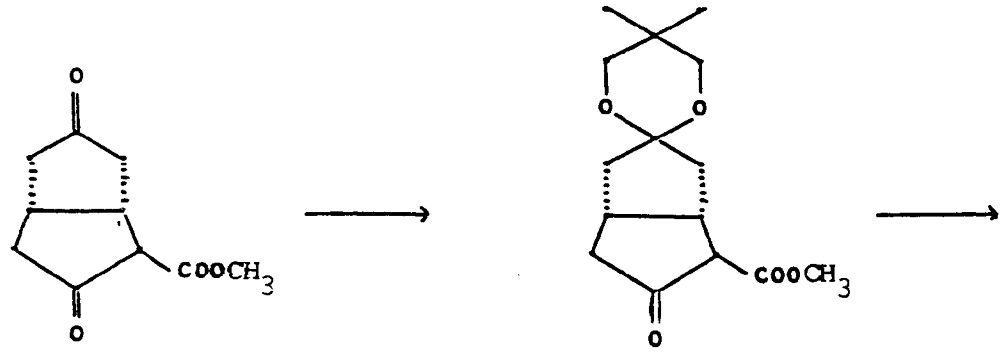

IIa IIb

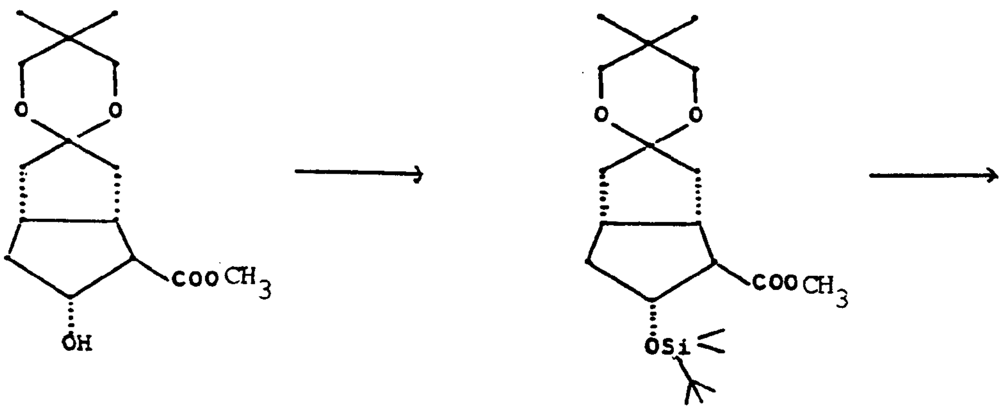

V VI

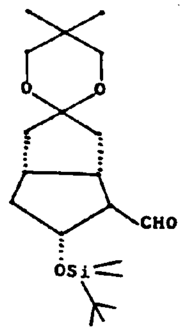

VII

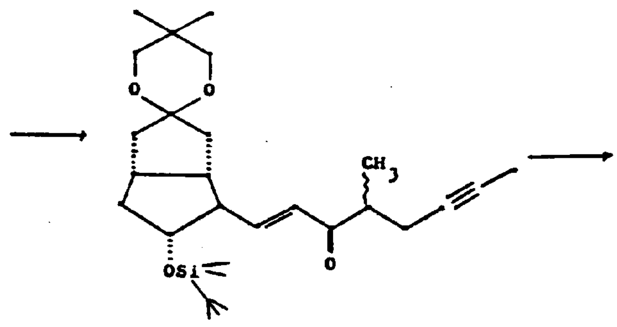

VIII

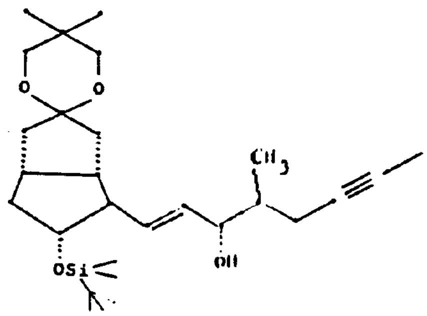

IX

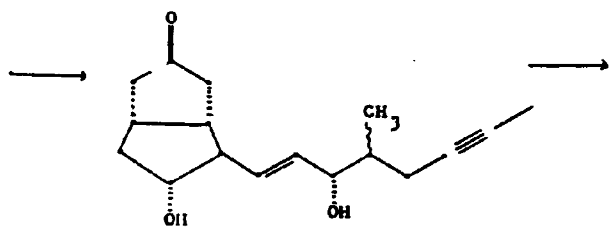

X

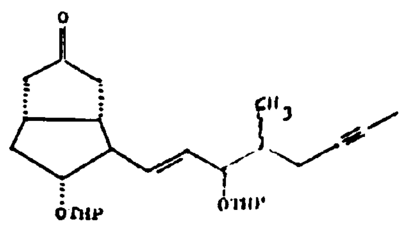

XI

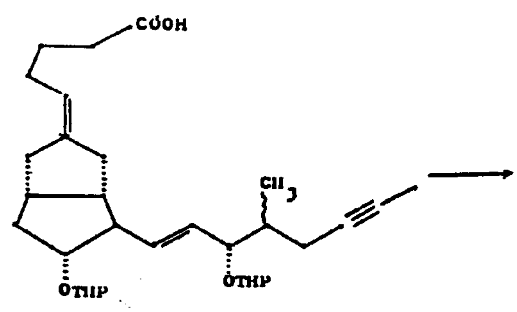

XII

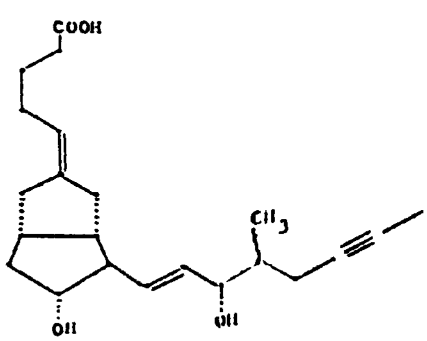

Iloprost

Nach regioselektivem Schutz der Carbonylgruppe in IIa mit Ameisensäureethylester in Gegenwart von 2,2-Dimethyl-1,3-diol und einer katalytischen Menge p-Toluolsulfonsäure wird IIb mit Natriumborhydrid in Ethanol

zum Alkohol V reduziert. Silylätherbildung (VI) und anschliessende Reduktion mit Diisobutylaluminiumhydrid in Toluol bei -70 °C liefert den Aldehyd VII, der mit 3-Methyl-2-oxo-hept-5-in-phosphonsäure-dimethylester und Natriumhydrid zum α,ß-ungesättigten Keton VIII kondensiert wird. Reduktion des Ketons VIII zum Alkohol IX, anschließende Schutzgruppenspaltung zum Diol X und Tetrahydropyranyletherbildung liefert das Keton XI, welches nach Wittig-Reaktion mit dem Ylen aus 4-Carboxybutyltriphenylphosphoniumbromid und anschließender Schutzgruppenabspaltung mit wäßriger Esigsäure in das Carbacyclinderivat Iloprost überführt wird.

Die folgenden Ausführungsbeispiele dienen zur Erläuterung des erfindungsgemäßen Verfahrens, ohne es jedoch zu begrenzen.

Beispiel 1

2,4-Bismethoxycarbonyl-bicyclo [3.3.0] octan-3,7-dion

Zu einer Suspension von 80,1 g Kaliumcarbonat in 670 ml Methanol fügt man bei 24 °C eine Lösung von 66 g 4-tert.-Butoxy-2-cyclopenten-1-on und 72,1 g Acetondicarbonsäureester in 165 ml Methanol und rührt 23 Stunden bei 25 °C unter Argon. Man säuert anschließend mit 30%iger Zitronensäurelösung auf pH 4 an, verdünnt mit Wasser und extrahiert fünfmal mit Methylenchlorid. Man wäscht die organische Phase dreimal mit Sole, trocknet über Natriumsulfat und dampft im Vakuum ein. Den Rückstand reinigt man durch Chromatographie an Kieselgel. Mit Essigester/Hexan-Gemischen eluiert man 64 g der Titelverbindung als farbloses Öl.
IR ($CHCl_3$): 3022, 2962, 1740, 1665, 1624, 1445/cm.

Das Ausgangsmaterial für die obige Titelverbindung wird wie folgt hergestellt:

1a)

4-tert.-Butoxy-2-cyclopenten-1-on

Zu einer Lösung von 64, 7 g 4-tert.-Butoxy-2-cyclopenten-1-ol (hergestellt aus Cyclopentadien gemäß: S. Takano et al., Heterocycles 16, 605 (1981)) in 750 ml Aceton tropft man bei -30 °C 90 ml Jones Reagenz (Chromtrioxid in Schwefelsäure) und rührt 20 Minuten bei -30 °C. Anschließend tropft man 50 ml Isopropylalkohol zu, verdünnt mit Äther und schüttelt mehrmals mit Wasser. Man trocknet mit Natriumsulfat und dampft im Vakuum ein. Der Rückstand wird ohne weitere Reinigung in die Kondensationsreaktion gemäß Beispiel 1 eingesetezt.

Beispiel 2

2,4-Bisethoxycarbonyl-bicyclo [3.3.0] octan-3,7-dion

Zu einer gerührten Suspension von 27 g Kaliumcarbonat in 116 ml Ethanol tropft man bei 0 °C unter Argon eine Lösung aus 13,7 4-Hydroxy-2-cyclopent-1-on (beispielsweise hergestellt aus 2-Methylfuran gemäß: v. Clauson-Kas et al., Acta chem. scand. 1947, 619 oder Synthesis 1986, 781) und 25,36 ml Acetondicarbonsäurediethylester in 42 ml Ethanol, rührt 1 Stunde bei 0 °C und 24 Stunden bei 25 °C. Man engt im Vakuum ein, fügt 200 ml Wasser zu, säuert mit einer 20%igen Zitronsäurelösung auf pH 4 an, extrahiert mit Methylenchlorid und wäscht den organischen Extrakt mit Sole. Man trocknet über Magnesiumsulfat und dampft im Vakuum ein. Den Rückstand reinigt man durch Chromatographie an Kieselgel. Mit Essigester/Hexan-Gemischen eluiert man 28 g der Titelverbindung als farbloses Öl.
IR ($CHCl_3$): 3021, 2960, 1740, 1665, 1624, 1446/cm.

Beispiel 3

2,4-Bismethoxycarbonyl-bicyclo [3.3.0] octan-3,7-dion

Zu einer Suspension von 900 mg Kaliumcarbonat in 7,5 ml Methanol fügt man bei 25 °C eine Lösung aus 1 g 4-Cumyloxy-2-cyclopenten-1-on und 0,83 g Acetondicarbonsäureester in 1,9 ml Methanol und rührt 24 Stunden bei 25 °C unter Argon. Man säuert anschließend mit 30%iger Zitronensäurelösung auf pH 4 an, verdünnt mit Wasser und extrahiert fünfmal mit Methylenchlorid. Man wäscht die organische Phase dreimal mit Sole, trocknet über Natriumsulfat und dampft im Vakuum ein. Den Rückstand reinigt man durch Chromatographie an Kieselgel. Mit Essigester/Hexan-Gemischen eluiert man 0,85 g der Titelverbindung als schwach rosa gefärbtes Öl.

IR ($CHCl_3$): 3021, 2960, 1740, 1665, 1624, 1446/cm.

Das Ausgangsmaterial für die obige Titelverbindung wird wie folgt hergestellt:

3a)

4-Cumyloxy-2-cyclopenten-1-on

Zu einer Lösung von 4,1 g 4-Cumyl-2-cyclopenten-1-ol (hergestellt aus Cyclopentadien gemäß S. Takano, Chem. Pharm. Bull. 34, 3445 (1986)) in 50 ml Aceton tropft man bei -30 °C 6 ml Jones-Reagenz und rührt 20 Minuten bei -30 °C. Anschließend tropft man 6 ml Isopropanol zu, verdünnt mit Äther und schüttelt mehrmals mit Wasser. Man trocknet mit Natriumsulfat und dampft im Vakuum ein. Nach Filtration des Rückstands über Kieselgel mit Hexan/Essigester-Gemischen erhält man 3,81 g 4-Cumyloxy-2-cyclopenten-1-on als farbloses Öl.

## Patentansprüche

Verfahren zur Herstellung von Bicyclo [3.3.0]-octandicarbonsäurediestern der allgemeinen Formel I,

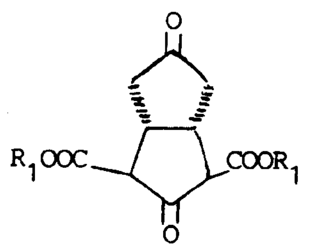

(I),

worin $R_1$ eine gerad- oder verzweigtkettige Alkylgruppe mit 1-10 C-Atomen darstellt, dadurch gekennzeichnet, daß man 2-Cyclopentenonderivate der allgemeinen Formel III,

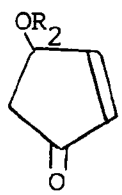

(III),

worin $R_2$ ein Waserstoffatom oder eine gerad- oder verzweigtkettige Alkylgruppe mit 1-10 C-Atomen, die gegebenenfalls durch eine Phenylgruppe substituiert sein kann, mit Acetondicarbonsäurediester der Formel IV,

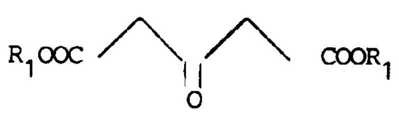

(IV),

worin $R_1$ die oben genannte Bedeutung hat, in Gegenwart einer Base umsetzt.

## Claims

Process for the preparation of bicyclo[3.3.0]octanedicarboxylic acid diesters of the general formula I

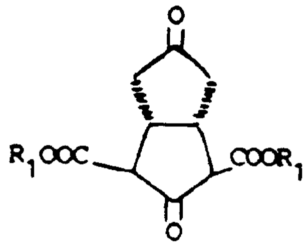

(I),

wherein $R_1$ represents a straight-chain or branched alkyl group having from 1 to 10 carbon atoms, characterised in that 2-cyclopentenone derivatives of the general formula III

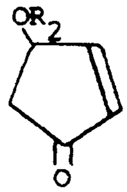

(III),

wherein $R_2$ represents a hydrogen atom or a straight-chain or branched alkyl group having from 1 to 10 carbon atoms which may optionally be substituted by a phenyl group, are reacted in the presence of a base with acetone-dicarboxylic acid diesters of formula IV

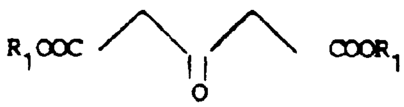

(IV),

wherein $R_1$ has the meaning given above.

**Revendications**

Procédé pour préparer des diesters de l'acide bicyclo[3.3.0]octane-dicarboxylique répondant à la formule I :

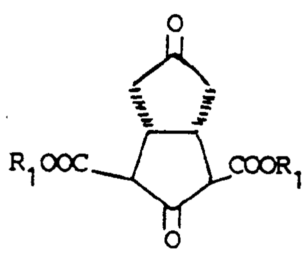

(I),

dans laquelle $R_1$ représente un radical alkyle, linéaire ou ramifié, qui contient de 1 à 10 atomes de carbone, procédé caractérisé en ce qu'on fait réagir, en présence d'une base, des dérivés de la cyclopenténone-2 répondant à la formule générale III :

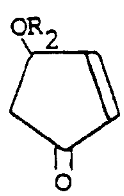

(III),

dans laquelle $R_2$ représente un atome d'hydrogène ou un radical alkyle, linéaire ou ramifié, qui contient de 1

à 10 atomes de carbone et qui peut éventuellement porter un radical phényle, avec un diester de l'acide acétone-dicarboxylique répondant à la formule IV :

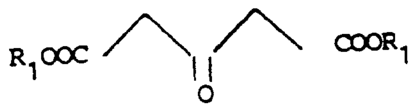

(IV),

dans laquelle $R_1$ a la signification qui lui a été donnée ci-dessus.